(19) Europäisches Patentamt
European Patent Office
Office européen des brevets

(11) Veröffentlichungsnummer: **0 235 647 B1**

(12) **EUROPÄISCHE PATENTSCHRIFT**

(45) Veröffentlichungstag der Patentschrift: **29.09.93**

(51) Int. Cl.⁵: **C07D 471/04**, C09B 48/00, //(C07D471/00,221:00,221:00)

(21) Anmeldenummer: **87102067.3**

(22) Anmeldetag: **13.02.87**

# (54) Verfahren zur Herstellung von 6,13-Dihydrochinacridonen und Chinacridonen.

(30) Priorität: **25.02.86 DE 3605976**

(43) Veröffentlichungstag der Anmeldung:
**09.09.87 Patentblatt 87/37**

(45) Bekanntmachung des Hinweises auf die Patenterteilung:
**29.09.93 Patentblatt 93/39**

(84) Benannte Vertragsstaaten:
**CH DE FR GB LI**

(56) Entgegenhaltungen:
**FR-A- 1 251 224**
**GB-A- 1 191 409**
**US-A- 2 821 530**
**US-A- 2 969 366**

**CHEMICAL REVIEWS, Band 67, Nr. 1, 25. Januar 1967, Seiten 1-18, American Chemical Society, Easton, US; S.S. LABANA et al.: "Ouinacridones"**

(73) Patentinhaber: **BAYER AG**

**D-51368 Leverkusen(DE)**

(72) Erfinder: **Herzog, Helmut, Dr.**
**Am Benthal 22**
**D-5090 Leverkusen 3(DE)**
Erfinder: **Schütze, Detlef-Ingo, Dr.**
**Roggendorfstrasse 51**
**D-5000 Köln 80(DE)**
Erfinder: **Schneider, Jürgen, Dr.**
**Am Thelensiefen 9**
**D-5068 Odenthal(DE)**
Erfinder: **Schmitz, Reinold, Dr.**
**Im Kerberich 36**
**D-5068 Odenthal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft ein Verfahren zur Herstellung eines 6,13-Dihydrochinacridons der allgemeinen Formel (I)

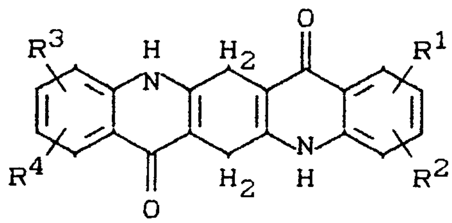

(I)

in der $R^1$, $R^2$, $R^3$, $R^4$ Wasserstoff oder F, Cl, Br, I, -OH, $-NO_2$, $-CF_3$, einen $C_1$-$C_4$-Alkylrest, einen $C_1$-$C_4$-Alkoxyrest, Phenyl, Cyclohexyl, Phenoxy, -COOH, -COO-$C_1$-$C_4$-Alkyl, $-SO_3H$,

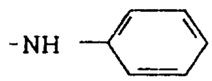

,

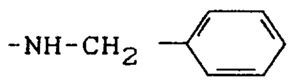

,

$-N(CH_3)_2$, $-SO_2NH_2$, $-SO_2N(CH_3)_2$, $-CONH_2$, $-CON(CH_3)_2$ oder

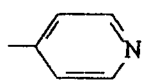

bedeuten, wobei
$R^1$ und $R^2$ (bzw. $R^3$ und $R^4$) auch zusammen einen ankondensierten Benzo- oder Naphthoring bilden können, dadurch gekennzeichnet, daß man einen 2,5-Di-(phenylamino)-3,6-dihydroterephthalsäuredialkyl-, vorzugsweise -dimethyl oder -diethylester, in einer im wesentlichen sauerstofffreien Atmosphäre in Gegenwart eines Dimethyldiphenylether-Isomerengemisches der Formel (II) oder (IIa) als Lösungs- und/oder Verdünnungsmittel auf Temperaturen von 240-320 °C erhitzt.

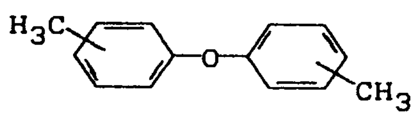

(II)

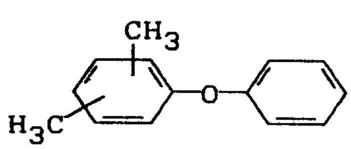

(IIa)

Bevorzugt eingesetzt wird das Isomerengemisch (II), das z.B. folgende Zusammensetzung haben kann:

| | |
|---|---|
| 0-5 | Gew.-% 2,2'-Dimethyldiphenylether |
| 5-40 | Gew.-% 2,3'- " |
| 5-30 | Gew.-% 2,4'- " |
| 10-50 | Gew.-% 3,3'- " |
| 10-50 | Gew.-% 3,4'- " |
| 0-20 | Gew.-% 4,4'- " |
| 0-5 | Gew.-% andere Komponenten. |

Eine bevorzugte Zusammensetzung für das Isomerengemisch ist:

| | |
|---|---|
| 0-3 | Gew.-% 2,2'-Dimethyldiphenylether |
| 10-30 | Gew.-% 2,3'- " |
| 10-20 | Gew.-% 2,4'- " |
| 20-35 | Gew.-% 3,3'- " |
| 15-35 | Gew.-% 3,4'- " |
| 0-10 | Gew.-% 4,4'- " |
| und | |
| 0-2 | Gew.-% andere Komponenten. |

Es ist aus der Literatur bereits bekannt, Dialkyl-2,5-diarylamino-3,6-dihydro-terephthalate mittels hochsiedender Ether im Temperaturbereich von 240-300°C zu cyclisieren, wobei die entsprechenden 6,13-Dihydrochinacridone erhalten werden.

So wird gemäß DAS 1 183 092, US-A-2 821 529 und 2 821 530 und GB-A-913 134 die Verwendung eines eutektischen Gemisches von Diphenyl und Diphenylether vorgeschlagen, während in JA 5 757 749 der Toyo Soda Mfg. der Einsatz von Dibenzylether als Lösungsmittel empfohlen wird. US-A-29 69 366 beschreibt ein Zyklisierungsverfahren in einem eutektischen Gemisch von Biphenyl und Diphenyloxid unter Erhalt von 6,13-Dihydrochinacridon. In GB-A-1 191 409 werden Dimethyldiphenylether als Mittel zum Wärmetransport und als Kühlmittel angegeben.

Hinsichtlich Ausbeute und Qualität könenn diese literaturbekannten Verfahren allerdings nicht überzeugen.

Wir fanden nun überraschenderweise, daß gemäß den Verfahren der Erfindung 6,13-Dihydrochinacridone (I) in ausgezeichneter Ausbeute und hoher Reinheit entstehen; sie dienen als wertvolle Zwischenprodukte zur Synthese von Chinacridon-Pigmenten.

Die als Ausgangsverbindungen verwendeten 2,5-Di-(phenylamino)-3,6-dihydro-terephthalsäuredialkylester sind bekannte Verbindungen, deren Synthese aus handelsüblichen Zwischenprodukten möglich ist.

Die Kondensation von Anilin oder dessen Derivaten mit Dialkylsuccinylsuccinat gegebenenfalls in Gegenwart eines Lösungsmittels bei erhöhter Temperatur und gegebenenfalls unter Druck ist säurekatalysiert, wobei als Säuren beispielsweise Anilinsalze, HCl, $H_2SO_4$, Essigsäure und p-Toluolsulfonsäure dienen können.

Als Lösungsmittel verwendet man z.B. Alkohole, wie Methanol oder Ethanol, Toluol, Xylol oder das Dimethylphenylether-Isomerengemisch (II).

Gegebenenfalls kann das (substituierte) Anilin gleichzeitig als Reaktionskomponente und Lösungsmittel dienen, zumal wenn es in großem Überschuß eingesetzt wird.

In Abhängigkeit von Lösungsmittel kann es nützlich sein, das bei der Kondensation gebildete Reaktionswasser durch Abdestillieren, beispielsweise am Wasserabscheider oder im Vakuum, zu entfernen.

Als Aniline benutzt man z.B. Anilin, m- und p-Chloranilin, m-und p-Toluidin und p-Anisidin, m- und p-Fluoranilin, m- und p-Bromanilin, m-und p-Nitroanilin, m- und p-Trifluormethylanilin, 4-Ethylanilin, 4-Cyclohexylanilin, Sulfanilsäure, 4-Aminobenzoesäure, 4-Aminobenzoesäuremethylester, 4-Phenylanilin, 3,4-oder 3,5-Dichloranilin, 3,4- oder 3,5-Dimethylanilin; besonders bevorzugt ist Anilin.

Die Verwendung von 2,5-Dianilino-3,6-dihydro-terephthalsäure-dimethylesterals Ausgangsmaterial für die Cyclisierung ist eine besonders bevorzugte erfindungsgemäße Verfahrensvariante.

Als Lösungs- und/oder Verdünnungsmittel ist das Dimethylphenylether-Isomerengemisch der allgemeinen Formel (II) besonders geeignet.

Die Überführung der Dialkyl-2,5-phenylamino-3,6-dihdyroterephthalate in die entsprechenden 6,13-Dihydrochinacridone in Gegenwart eines Isomerengemisches der Formel (II) bzw. (IIa) geschieht im

Temperaturbereich von 240-320 °C, vorzugsweise bei 280-290 °C, unter Inertgas-Atmosphäre, wobei als Inertgas z.B. Stickstoff, Kohlendioxid und Argon verwendet werden können.

Eine bevorzugte Ausführungsform des erfindungsgemäßen Verfahrens besteht darin, daß man eine 100-150 °C heiße Suspension aus 2,5-Dianilino-3,6-dihydro-terephthalatsäuredimethyl- oder -ethylester und dem Isomerengemisch (II) bzw. (IIa) innerhalb 15-90 Minuten unter Stickstoff in siedendes Isomerengemisch (II) bzw. (IIa) eindosiert und den Ringschluß durch 15-60 minütiges Rückflußerhitzen unter Abdestillieren von Methanol bzw. Ethanol zu Ende bringt.

Pro Gewichtsteil 2,5-Dianilinodihydrodiester setzt man vorzugszweise 3-20 Volumenteile, besonders bevorzugt 5-12 Volumenteile des Isomerengemischs (II) bzw. (IIa) ein.

Die Herstellung der unsubstituierten 2,5-Dianilino-3,6-dihdyro-dialkylester und der nachfolgende Ringschluß zum 6,13-Dihydrochinacridon im Isomerengemisch (II) bzw.(IIa) kann vorteilhafterweise auch als Eintopfreaktion durchgeführt werden.

Zu diesem Zweck kondensiert man z.B. Succinylobernsteinsäure-dialkylester mit mindestens 2 Mol, vorzugsweise 4-8 Mol Anilin im Isomerengemisch (II) bzw. (IIa) als Lösungs-und/oder Verdünnungsmittel in Gegenwart eines sauren Katalysators, vorzugsweise wäßriger Salzsäure, bei 90-130 °C und 20-120 mbar und neutralisiert anschließend mit wäßriger Sodalösung.

Nachfolgend befreit man das Reaktionsgemisch durch Vakuum-destillation von Wasser und überschüssigem Anilin und verdünnt gegebenenfalls mit Isomerengemisch (II) bzw. (IIa).

Die aus 2,5-Dianilin-3,6-dihydro-terephthalsäure-dialkylester und Isomerengemisch (II) bzw. (IIa) bestehende Suspension wird dann, wie oben angegeben, zum 6,13-Dihydrochinacridon ringgeschlossen.

Nach Cyclisierung im Isomerengemisch (II) bzw. (IIa) wird eine Suspension des gegebenenfalls substituierten 6,13-Dihydrochinacridons im Isomerengemisch erhalten, die in üblicher Weise aufgearbeitet wird. Z.B. kann man bei Temperaturen (150 °C absaugen und den anfallenden Nutschkuchen durch Waschen mittels Alkohol, vorzugsweise Methanol von anhaftenden Lösungsmitteln und Nebenprodukten befreien und anschließend gegebenenfalls nochmals in heißem Methanol ausrühren, um Reste des Isomerengemischs (II) bzw. (IIa) zu entfernen.

Das Eintopfverfahren erfordert bei der Aufarbeitung neben der Alkohol- noch eine Wasserwäsche zur Beseitigung enthaltener Salze.

Alle bei dem Cyclisierungsverfahren anfallenden Abfälle (Mutterlaugen, Waschlaugen) können problemlos in hoher Ausbeute destillativ auf Isomerengemisch (II) bzw. (IIa) und Methanol aufgearbeitet werden, während die Rückstände in flüssiger Form verbrannt werden.

Die Synthese der gegebenenfalls substituierten 6,13-Dihydrochinacridone, ausgehend von 2,5-Dianilino-3,6-dihydroterephthalsäuredialkylestern durch Ringschlußreaktion im Isomerengemisch (II) bzw. (IIa) verläuft in hohen Ausbeute.

Bei allen vorstehend genannten Reaktionen wird vorzugsweise das Isomerengemisch (II) eingesetzt.

Für das Eintopfverfahren, das von den substituierten Succinylbernsteinsäure-dialkylestern ausgeht, erhält man ebenfalls hohe Ausbeuten.

Die erfindungsgemäß herstellten Produkte fallen sehr rein an, wie IR- und UV-spektroskopische Daten zeigen; sie können als methanol- oder wasserhaltige Pasten oder aber in trockener Form weiterverarbeitet werden.

Die Oxidation von 6,13-Dihydrochinacridonen ist prinzipiell bekannt (z.B. gemäß US 2 821 529, Beispiele 9-15; GB 909 602, Beispiele 1-6; GB 911 477, Beispiele 1-11).

Die Verbindungen der allgemeinen Formel (I), synthetisiert gemäß dem Verfahren der Erfindung, können z.B. durch Oxidation mittels m-nitrobenzolsulfonsaurem Natrium, Nitrobenzol, Nitronaphthalin, Nitrobenzolsulfonsäure und -carbonsäure, Nitrophenole, Sauerstoff oder Luft im Lösungsmittelgemischen aus Methanol, Ethanol, Aceton oder Ethylenglykol bzw. Glykolethern und wasser in Gegenwart von Alkali bei erhöhter Temperatur und gegebenenfalls unter Druck und gegebenenfalls in Anwesenheit von Dispergiermitteln und Reaktionsbeschleunigern in die entsprechenden Chinacridone umgewandelt werden. Bevorzugt wird mit Luft in Gegenwart eines Dispergiermittels, vorzugsweise eines anionischen Dispergiermittels, z.B. eines Kondensationsproduktes aus einer aromatischen Sulfonsäure und Formaldehyd, oxidiert.

Die Isolierung der tiefrotgefärbten Chinacridone geschieht üblicherweise durch Filtration, gefolgt von einer Alkohol-(gegebenenfalls) und Wasserwäsche. Nach Trocknen muß das Rohpigment eventuell durch geeignete Formierung (z.B. Salzmahlung) optimal feinverteilt werden.

Erfindungsgemäß lassen sich beispielsweise die folgenden Chinacridone synthetisieren:
$\beta$- und $\gamma$-Chinacridon, 2,9-Dichlorchinacridon, 3,10-Dichlorchinacridon, 2,9-Dimethylchinacridon, 2,9-Dimethoxychinacridon, 2,9-Diethoxychinacridon, 2,4,9,11-Tetrachlorchinacridon, 2,9-Dicyclohexylchinacridon, 2,9-Diphenylchinacridon, 3,10-Dinitrochinacridon, 1,2,8,9-Tetrachlorchinacridon, 2,9-Difluorchinacridon und 2,9-Dibromchinacridon.

Die Erfindung betrifft weiterhin ein Verfahren zur Herstellung von γ-Chinacridon, das dadurch gekennzeichnet ist, daß man das nach den vorstehend beschriebenen Verfahren erhaltene 6,13-Dihydrochinacridon mit einem im wesentlichen wasserfreien Gemisch aus einem $C_1$-$C_4$-Alkohol und Alkali behandelt, und man anschließend das Dihydrochinacridon wie oben angegeben in üblicher Weise, vorzugsweise mit Luft, oxidiert. Vorzugsweise setzt man pro Gewichtsteil 6,13-Dihydrochinacridon 3-10, vorzugsweise 4-6 Gewichtsteile Alkohol, 0,3-1, vorzugsweise 0,3-0,6 Gewichtsteil Alkali und 0-0,5, vorzugsweise 0-0,1 Gewichtsteil Wasser ein. Als Alkohol wird vorzugsweise Methanol verwendet. Als Alkali wird vorzugsweise Natriumhydroxid eingesetzt.

Die Behandlung der 6,13-Dihydroverbindung kann z.B. in der Weise erfolgen, daß man das Reaktionsgemisch 15-60 Minuten zum Sieden erhitzt oder mehrere Stunden bei Raumtemperatur rührt.

In der Regel erfolgt durch die Behandlung mit dem Alkohol-Alkali-Gemisch eine Umwandlung von α- in β-6,13-Dihydrochinacridon. Das nach dem neuen Verfahren erhaltene γ-Chinacridon zeichnet sich durch hohe Reinheit, hohes Deckvermögen, hohen Glanz und weitere hervorragende koloristische Eigenschaften aus.

Anhand der nun folgenden Beispiele soll das erfindungsgemäße Verfahren näher erläutert werden.

Beispiel 1

Ein Gemisch aus 600 ml Methanol, 34,3 ml Eisessig, 95,9 ml Anilin und 100 g Dimethylsuccinylsuccinat erhitzt man in einem 1,2 l-VA-Autoklaven innerhalb 30 Minuten auf 100 °C und hält 6 h bei 100-105 °C.

Nach Abkühlen entspannt man den Autoklaven und entnimmt dem Reaktionsgemisch eine Probe, die dünnschichtchromatographisch < 3 % an Ausgangsmaterial enthielt.

Die erhaltene Suspension wird abgesaugt, nacheinander mit Methanol und Wasser gewaschen und bei 70-80 °C im Vakuum getrocknet. Erhalten wurden 160 g ≙ 96,5 % eff eines schwach orangerot gefärbten Produktes, das nach HFC 96-98 %ig ist, ein charakteristisches IR-Spektrum zeigt und durch die Formel (III) wiedergegeben wird:

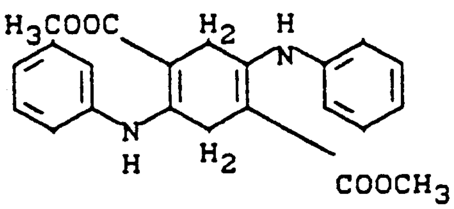

(III)

1a) Ersetzt man Anilin durch 134 g p-Chlor- oder m-Chloranilin, arbeitet man ansonsten wie oben angegeben, so gewinnt man in Effektivausbeuten von 96,7 % ≙ 189,5 g und 95 5 % ≙ 186,2 g Produkte, die 15,95 bzw. 15,7 % Chlor enthalten, charakteristische IR-Spektren zeigen und den Formeln (IV) und(V) entsprechen:

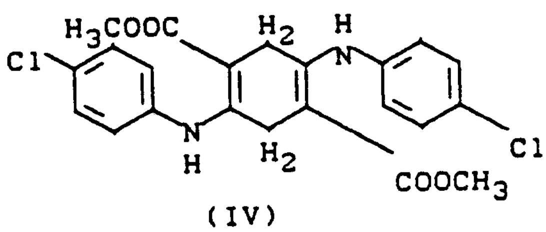

(IV)

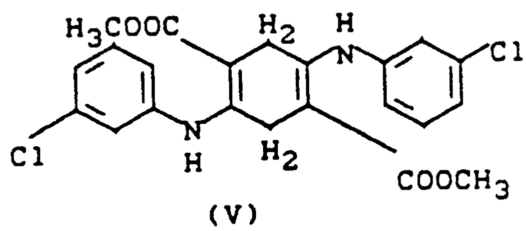

(V)

1b) Ersetzt man Anilin durch 112,4 g p-Toluidin, arbeitet ansonsten wie in Beispiel 1 angegeben, so werden 173,4 g = 97,3 % eff einer Verbindung erhalten, die charakteristische IR-Banden zeigt und der folgenden Formel entspricht:

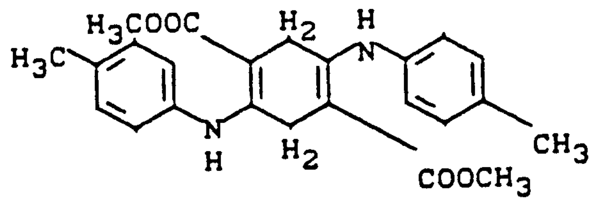

Beispiel 2

Eine Suspension aus 80 g 2,5-Dianilino-3,6-dihydro-terephtalsäuredimethylester, hergestellt gemäß Beispiel 1, in 350 ml eines Gemisches aus (3 Gew.-% 2,2'-, 10-30 Gew.-% 2,3'-, 10-20 Gew.-% 2,4'-, 20-35 Gew.-% 3,3'-, 15-35 Gew.-% 3,4'- und <10 Gew.-% 4,4'-Dimethyldiphenylether erhitzt man unter Rühren ($N_2$-Atmosphäre) in einem 1 l-Planschliffbecher mit Bodenablaßventil auf 100-150 °C.

Anschließend wird diese heiße Suspension innerhalb 45-60 Minuten in 450 ml siedendes Isomerengemisch der im vorstehenden Absatz beschriebenen Zusammensetzung (Temperatur: 280-290 °C, Rührgeschwindigkeit: 200 U/Min) unter $N_2$ eindosiert und nachfolgend hält man das Reaktionsgemisch noch 20-30 Minuten bei 284-7 °C (Rückfluß).

Die Reaktion beginnt schon nach wenigen Minuten unter Methanol-Entwicklung (insgesamt werden ca. 15-16 ml Dimethyldiphenylether-haltiges Methanol durch Abdestillieren gewonnen), verbunden mit einsetzender Dihydrochinacridon-Bildung in Form eines orangefarbenen Niederschlags (starkes Schäumen).

Nach Abkühlen auf 20-30 °C wird die erhaltene Suspension über eine 1l-G3-Glasfilternutsche scharf abgesaugt und 5 x mit je 100 ml Methanol gewaschen, worauf der Ablauf noch schwach gelb gefärbt ist.

Anschließend erhitzt man den Nutschkuchen kurz mit 400 ml Methanol unter gutem Rühren zum Sieden, saugt ab, wäscht mit ca. 300 ml Methanol bis zum farblosen Ablauf und trocknet bei 70-80 °C im Vakuum.

Erhalten werden 62,5 g ≙ 94,1 % eff blaßrosafarbenes 6,13-Dihydrochinacridon der Formel (VI)

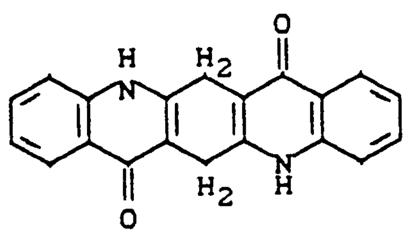

(VI)

das nach IR- und UV-Spektrum sehr rein ist.

2a) Reduziert man die Isomerengemischmenge auf 600 ml (= 7,5 Volumenteile pro Gewichtsteil 2,5-Dianilino-3,6-dihydro-terephthalsäuredimethylester), arbeitet ansonsten wie oben angegeben, so beträgt die Ausbeute an reinem 6,13-Dihydrochinacridon 60,9 g ≙ 91,6 % eff.

Beispiel 3

Ein Reaktionsgemisch aus 50 g Dimethylsuccinylsuccinat (= DMSS), 250 ml des in Beispiel 1 angegebenen Dimethyldiphenylether-Isomerengemischs, 150 ml Anilin und 0,5 ml 30 %ige Salzsäure läßt man zunächst ca 30 Minuten bei 20-30 °C rühren und erwärmt dann innerhalb 1 h unter gutem Rühren bei 70 mm Hg Vakuum auf 105-110 °C. Anschließend hält man 3 h bei 105-110 °C, wobei 9-12 ml Wasser/Anilin-Gemisch abdestillieren. Dünnschichtchromatographisch ist nach dieser Zeit praktisch kein DMSS mehr nachzuweisen.

Nach Abkühlen auf 50 °C wird belüftet und unter Stickstoff 0,3 g wasserfreie Soda, gelöst in 10 ml Wasser, zugefügt und 1 h bei 50-60 °C gerührt.

Nachfolgend destilliert man bei 15-30 mm Hg Wasserstrahlvakuum 170 ml Wasser/Anilin/Dimethylphenylether-Isomerengemisch ab; die Reaktionssuspension, bestehend aus 2,5-Dianilino-3,6-dihydro-terephthalsäuredimethylester und Isomerengemisch ist praktisch anilinfrei.

Nach Abkühlen und Belüften verdünnt man die Reaktionsmischung mit 200 ml des Dimethyldiphenylether-Isomerengemischs, erhitzt unter Stickstoff und gutem Rühren auf 100-150 °C und dosiert die heiße Suspension unter $N_2$ innerhalb 45-60 Minuten in 600 ml siedendes Isomerengemisch ein und hält anschließend noch 20-30 Minuten bei 284-7 °C (Rückfluß).

Die Aufarbeitung vollzieht sich wie im Beispiel 2 angegeben wurde, wobei zusätzlich vor dem Trocknen noch mit Wasser gewaschen wurde.

Erhalten werden 60,5 g = 87,8 % eff (bezogen auf eingesetztes DMSS) reines 6,13-Dihydrochinacridon (VI).

Beispiel 4

Arbeitet man wie in Beispiel 2 angegeben, verwendet aber die gemäß Beispiel 1a hergestellte Verbindung (IV) als Ausgangsmaterial, so erhält man 63 g = 91,9 % eff reines 2,9-Dichlor-6,13-dihydrochinacridon der Formel (VII)

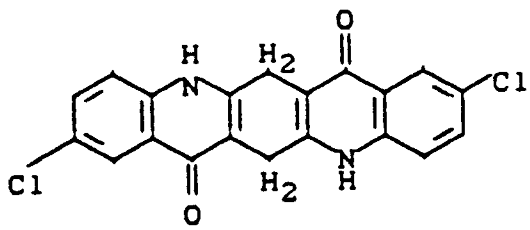

(VII)

Beispiel 4a

Arbeitet man gemäß Beispiel 2, setzt aber das nach Beispiel 1b synthetisierte Produkt ein, so werden 59,6 g = 88,4 % eff reines 2,9-Dimethyl-6,13-dihydrochinacridon der Formel (VIII) erhalten

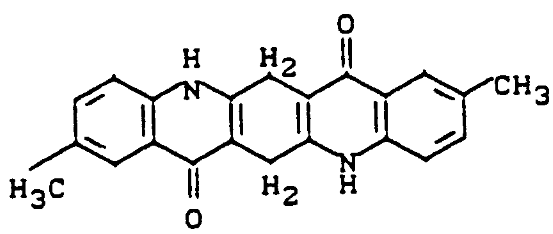

(VIII)

Beispiel 5

In einem 1l-Planschliffbecher mit Ankerrührer löst man 12 g NaOH rotuli in 140 ml Methanol unter gutem Rühren und trägt dann 30 g 6,13-Dihydrochinacridon, hergestellt gemäß Beispiel 2, ein.

Nach Verdünnen mit 60 ml Methanol, rührt man 10 Minuten bei 20-30 °C nach und erhitzt nachfolgend mittels eines Ölbades 1 h am Rückfluß.

Nun versetzt man mit einer 65 °C heißen Lösung aus 3 g eines handelsüblichen Dispergiermittels, z.B. auf Basis eines Kondensationsprodukts einer aromatischen Sulfonsäure mit Formaldehyd, in 220 ml dest. Wasser und erwärmt weitere 30 Minuten zum Rückfluß.

Anschließend wird nach Zugabe von 0,75 g anthrachinon-2-sulfonsaurem Natrium während 10-15 h unter Rückfluß erhitzt (70-80 °C) und 10-15 l Luft/h über ein Rotameter durch die Suspension geleitet, wobei die Farbe allmählich von blaßrosafarben nach tiefrot umschlägt.

Der Fortgang der Reaktion wird durch Entnahme von Proben UV-spektroskopisch kontrolliert.

Nach vollständiger Umsetzung saugt man heiß über eine 1/2 l G4-Fritte ab und wäscht den Nutschkuchen mit heißem Wasser bis zum farblosen Ablauf neutral.

Anschließend wird der Nutschkuchen in 400 ml dest. Wasser angeschlagen, mit ~ 1,5 ml 50 %iger Schwefelsäure kongosauer gestellt und ~ 30 Minuten bei ca 80 °C gerührt.

Nach Absaugen wäscht man den Filterkuchen mit heißem, dest. Wasser neutral und trocknet bei 60-80 °C.

Erhalten werden 29 g = 97,3 % eff reines γ-Chinacridon-Pigment, das im Lackaufstrich ein farbstarkes Rot mit hohem Deckvermögen, hohem Glanz, großer Klarheit und sehr gutem Fließverhalten darstellt.

## Beispiel 6

Ein Gemisch aus 240 ml Methanol und 60 g NaOH wird solange gerührt, bis fast alles gelöst ist.

Nach Eintragen von 30 g 6,13-Dihydrochinacridon, hergestellt gemäß Beispiel 2, verdünnt man mit einer Lösung von 3 g eines handelsüblichen Dispergiermittels in 180 ml Wasser und rührt dann noch 30 Minuten bei 20-30 °C nach.

Unter Einleiten von 10-15 l Luft/h wird nachfolgend 10-15 h unter Rückfluß erhitzt, wobei der Fortgang der Reaktion durch Entnahme von Proben UV-spektroskopisch kontrolliert wird.

Nach vollständiger Umsetzung wird bei Siedetemperatur mit 500 ml 60-65 °C warmem Wasser verdünnt und noch 1 h bei 65-70 °C nachgerührt.

Die erhaltene Suspension saugt man ab, wäscht den Nutschkuchen mit heißem Wasser neutral und trocknet bei 70-80 °C im Vakuum.

Erhalten werden 28 g ≙ 93,9 % eff eines Produktes, das nach Formierung, beispielsweise einer Salzmahlung in lasierende oder deckende β-Chinacridon-Pigmente (Nuance; Violett) von hoher Reinheit überführt werden kann.

## Beispiel 7

Ein Gemisch aus 155 ml Methanol, 45 ml Wasser und 24,8 g Kaliumhydroxid löst man bei 50-60 °C unter Rühren und tragt dann bei 20-30 °C innerhalb ca. 15 Minuten 27,5 g 2,9-Dichlordihydrochinacridon, hergestellt gemäß Beispiel 4, ein.

Anschließend erwärmt man das Reaktionsgemisch auf 40-50 °C und bringt es zusammen mit 35,8 g m-nitrobenzolsulfonsaurem Natrium in einen 0,7 l-VA-Autoklaven ein und erhitzt nachfolgend 5 h bei 100 °C.

Eine nach dieser Zeit entnommene und aufgearbeitete Probe weist UV-spektroskopisch <5 % an Ausgangsmaterial auf. Nach vollständiger Umsetzung entspannt und entleert man den Autoklaven, setzt 300 ml dest. Wasser zu und rührt die erhaltene Suspension noch 30 Minuten bei ~65 °C nach.

Nun saugt man heiß ab, wäscht mit heißem dest. Wasser neutral bis zum farblosen Ablauf und trocknet bei 70 °C im Vakuum. Erhalten werden 25,5 g = 93,2 % eff einer Verbindung, die nach Formierung, z.B. einer Salzmahlung, in lasierende oder deckende, magentafarbene 2,9-Dichlorchinacridon-Pigmente von hoher Reinheit umgewandelt werden kann.

## Beispiel 8

Eine Mischung, bestehend aus 22 g KOH/45 ml Wasser dest. (Lösung), 24,6 g 2,9-Dimethyldihydrochinacridon, hergestellt gemäß Beispiel 4a, und 38 g m-nitrobenzolsulfonsaurem Natrium wird in einen 0,7 l-VA-Autoklaven gegeben und 8 h bei 100-105 °C erhitzt.

Nach dieser Zeit ist die Oxidation praktisch abgeschlossen, wie sich UV-spektroskopisch nachweisen läßt.

Die Aufarbeitung gemäß Beispiel 7 führt zu einem Produkt (23,8 g ≙ 97,3 % eff), das durch Formierung, beispielsweise mittels Salzmahlung, in lasierende oder deckende blaustichig rote 2,9-Dimethylchinacridon-Pigmente von hoher Reinheit umgewandelt werden kann.

## Patentansprüche

1. Verfahren zur Herstellung eines 6,13-Dihydrochinacridons der allgemeinen Formel (I)

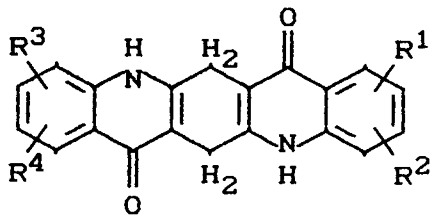

(I)

in der
$R^1$, $R^2$, $R^3$, $R^4$ Wasserstoff oder F, Cl, Br, I, -OH, $-NO_2$, $-CF_3$, einen $C_1$-$C_4$-Alkylrest, einen $C_1$-$C_4$-Alkoxyrest, Phenyl, Cyclohexyl, Phenoxy, -COOH, -COO-$C_1$-$C_4$-Alkyl, $-SO_3H$,

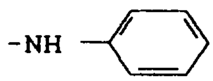

,

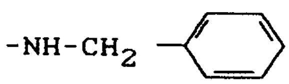

,

$-N(CH_3)_2$, $-SO_2NH_2$, $-SO_2N(CH_3)_2$, $-CONH_2$, $-CON(CH_3)_2$ oder

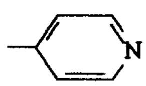

bedeuten, wobei $R^1$ und $R^2$ bzw. $R^3$ und $R^4$ zusammen einen ankondensierten Benzo- oder Naphthoring bilden können,
dadurch gekennzeichnet, daß man einen 2,5-Di-(phenylamino)-3,6-dihydro-terephthalsäuredialkylester in einer im wesentlichen sauerstofffreien Atmosphäre in Gegenwart eines Dimethyldiphenylether-Isomerengemisches der Formel

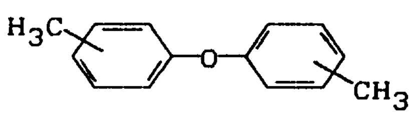

(II)

und/oder

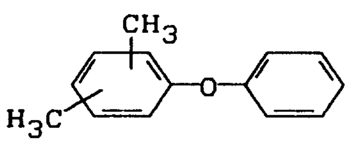

(IIa)

als Lösungs- und/oder Verdünnungsmittel auf Temperaturen von 240-320 °C erhitzt.

2. Verfahren gemäß dem Anspruch 1, dadurch gekennzeichnet, daß man in Gegenwart eines Isomerengemisches (II) aus 0-5 Gew.-% 2,2'-Dimethyldiphenylether, 5-40 Gew.-% 2,3'-Dimethyldiphenylether, 5-30 Gew.-% 2,4'-Dimethyldiphenylether, 10-50 Gew.-% 3,3'-Dimethyldiphenylether, 10-50 Gew.-% 3,4'-Dimethyldiphenylether, 0-20 Gew.-% 4,4'-Dimethyldiphenylether und 0-5 Gew.-% andere Komponenten arbeitet.

**3.** Verfahren gemäß den Ansprüchen 1-2, dadurch gekennzeichnet, daß man in Gegenwart eines Isomerengemisches (II) aus 0-30 Gew.-% 2,2'-Dimethyldiphenylether, 10-30 Gew.-% 2,3'-Dimethyldiphenylether, 10-20 Gew.-% 2,4'-Dimethyldiphenylether, 20-35 Gew.-% 3,3'-Dimethyldiphenylether, 15-35 Gew.-% 3,4'-Dimethyldiphenylether, 0-10 Gew.-% 4,4'-Dimethyldiphenylether und 0-20 Gew.-% andere Komponenten arbeitet.

**4.** Verfahren gemäß den Ansprüchen 1-3, dadurch gekennzeichnet, daß man einen 2,5-Di-(phenylamino)-3,6-dihydroterephthalsäuredimethyl-oder -diethylester einsetzt.

**5.** Verfahren gemäß den Ansprüchen 1-4, dadurch gekennzeichnet, daß man auf 280-290 °C erhitzt.

**6.** Verfahren gemäß den Ansprüchen 1-5, dadurch gekennzeichnet, daß man pro Gewichtsteil 2,5-Di-(phenylamino)-3,6-dihydro-terephthalsäuredialkylester 3-20, bevorzugt 5-12 Volumenteile Isomerengemisch (II) bzw. (IIa) einsetzt.

**7.** Verfahren zur Herstellung eines Chinacridons, dadurch gekennzeichnet, daß man das gemäß den Ansprüchen 1-6 erhaltene 6,13-Dihydrochinacridon in üblicher Weise oxidiert.

**8.** Verfahren zur Herstellung von $\gamma$-Chinacridon, dadurch gekennzeichnet, daß man gemäß den Ansprüchen 1-6 erhaltenes 6,13-Dihydrochinacridon mit einem im wesentlichen wasserfreien Gemisch aus einem $C_1$-$C_4$-Alkohol, vorzugsweise Methanol, und Alkali, vorzugsweise NaOH, behandelt, und man anschließend oxidiert.

**9.** Verfahren gemäß den Ansprüchen 7 und 8, dadurch gekennzeichnet, daß man mit Luft oxidiert.

**Claims**

**1.** Process for the preparation of a 6,13-dihydroquinacridone of the general formula (I)

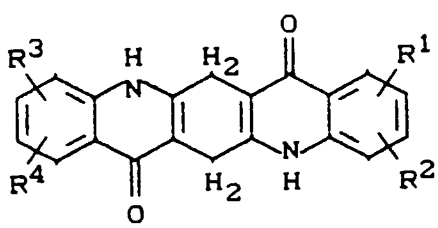

(I)

in which

$R^1$, $R^2$, $R^3$ and $R^4$ denote hydrogen or F, Cl, Br, I, -OH, $-NO_2$, $-CF_3$, a $C_1$-$C_4$-alkyl radical, a $C_1$-$C_4$-alkoxy radical, phenyl, cyclohexyl, phenoxy, -COOH, -COO-$C_1$-$C_4$-alkyl, $-SO_3H$,

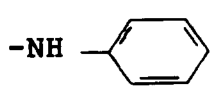 , 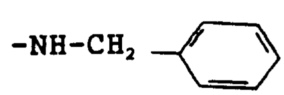 ,

$-N(CH_3)_2$, $-SO_2NH_2$, $-SO_2N(CH_3)_2$, $-CONH_2$, $-CON(CH_3)_2$ or

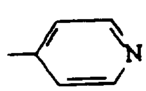 ,

it being possible for $R^1$ and $R^2$ or $R^3$ and $R^4$ together to form a fused-on benzo or naphtho ring,

characterised in that a dialkyl 2,5-di-(phenylamino)-3,6-dihydro-terephthalate is heated to temperatures of 240-320 °C in an essentially oxygen-free atmosphere in the presence of a dimethyldiphenyl ether isomer mixture of the formula

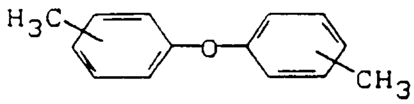

(II)

and/or

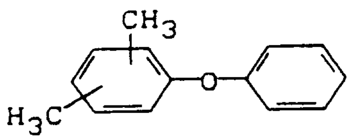

(IIa)

as the solvent and/or diluent.

2. Process according to Claim 1, characterised in that it is carried out in the presence of an isomer mixture (II) of 0-5 % by weight of 2,2'-dimethyldiphenyl ether, 5-40 % by weight of 2,3'-dimethyldiphenyl ether, 5-30 % by weight of 2,4'-dimethyldiphenyl ether, 10-50 % by weight of 3,3'-dimethyldiphenyl ether, 10-50 % by weight of 3,4'-dimethyldiphenyl ether, 0-20 % by weight of 4,4'-dimethyldiphenyl ether and 0-5 % by weight of other components.

3. Process according to Claims 1-2, characterised in that it is carried out in the presence of an isomer mixture (II) of 0-30 % by weight of 2,2'-dimethyldiphenyl ether, 10-30 % by weight of 2,3'-dimethyldiphenyl ether, 10-20 % by weight of 2,4'-dimethyldiphenyl ether, 20-35 % by weight of 3,3'-dimethyldiphenyl ether, 15-35 % by weight of 3,4'-dimethyldiphenyl ether, 0-10 % by weight of 4,4'-dimethyldiphenyl ether and 0-20 % by weight of other components.

4. Process according to Claims 1-3, characterised in that a dimethyl or diethyl 2,5-di-(phenylamino)-3,6-dihydroterephthalate is employed.

5. Process according to Claims 1-4, characterised in that the mixture is heated to 280-290 °C.

6. Process according to Claims 1-5, characterised in that 3-20, preferably 5-12, parts by volume of isomer mixture (II) or (IIa) are employed per part by weight of dialkyl 2,5-di-(phenylamino)-3,6-dihydroterephthalate.

7. Process for the preparation of a quinacridone, characterised in that the 6,13-dihydroquinacridone obtained according to Claims 1-6 is oxidised in the customary manner.

8. Process for the preparation of γ-quinacridone, characterised in that 6,13-dihydroquinacridone obtained according to Claims 1-6 is treated with an essentially anhydrous mixture of a $C_1$-$C_4$-alcohol, preferably methanol, and an alkali, preferably NaOH, and the mixture is subsequently oxidised.

9. Process according to Claims 7 and 8, characterised in that the oxidation is carried out with air.

## Revendications

1. Procédé de préparation d'une 6,13-dihydroquinacridone de formule générale I

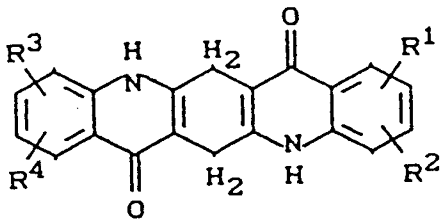

(I)

dans laquelle $R^1$, $R^2$, $R^3$, $R^4$ représentent l'hydrogène ou F, Cl, Br, I, -OH, $-NO_2$, $-CF_3$, un groupe alkyle en $C_1$-$C_4$, alcoxy en $C_1$-$C_4$, phényle, cyclohexyle, phénoxy, -COOH, -COO-alkyle en $C_1$-$C_4$, $-SO_3H$,

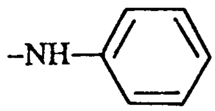
,

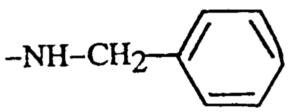
,

$-N(CH_3)_2$, $-SO_2NH_2$, $-SO_2N(CH_3)_2$, $-CONH_2$,

$-CON(CH_3)_2$ ou 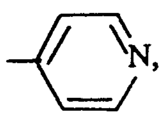,

$R^1$ et $R^2$ (d'une part, $R^3$ et $R^4$, d'autre part pouvant également former un cycle benzo ou naphto condensé,
caractérisé en ce que l'on chauffe à des températures de 240 à 320 °C un 2,5-di-(phénylamino)-3,6-dihydrotéréphtalate de dialkyle, en atmosphère essentiellement exempte d'oxygène, en présence d'un mélange d'éthers diméthyldiphényliques isomères de formules

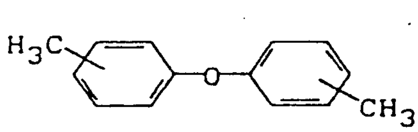

(II)

et/ou

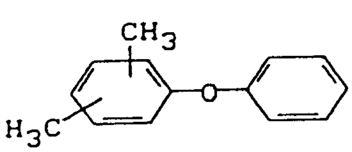

(IIa)

qui sert de solvant et/ou diluant.

**2.** Procédé selon revendication 1, caractérisé en ce que l'on opère en présence d'un mélange d'isomères II consistant en 0 à 5 % en poids d'éther 2,2'-diméthyldiphénylique, 5 à 40 % en poids d'éther 2,3'-diméthyldiphénylique, 5 à 30 % en poids d'éther 2,4'-diméthyldiphénylique, 10 à 50 % en poids d'éther 3,3'-diméthyldiphénylique, 10 à 50 % en poids d'éther 3,4'-diméthyldiphénylique, 0 à 20 % en poids d'éther 4,4'-diméthyldiphénylique et 0 à 5 % en poids d'autres composants.

**3.** Procédé selon les revendications 1 et 2, caractérisé en ce que l'on opère en présence d'un mélange d'isomères II consistant en 0 à 30 % en poids d'éther 2,2'-diméthyldiphénylique, 10 à 30 % en poids d'éther 2,3'-diméthyldiphénylique, 10 à 20 % en poids d'éther 2,4'-diméthyldiphénylique, 20 à 35 % en poids d'éther 3,3'-diméthyldiphénylique, 15 à 35 % en poids d'éther 3,4'-diméthyldiphénylique, 0 à 10 % en poids d'éther 4,4'-diméthyldiphénylique et 0 à 20 % en poids d'autres composants.

**4.** Procédé selon les revendications 1 à 3, caractérisé en ce que l'on part d'un 2,5-di-(phénylamino)-3,6-dihydrotéréphtalate de diméthyle ou de diéhyle.

**5.** Procédé selon les revendications 1 à 4, caractérisé en ce que l'on chauffe à 280-290 °C.

**6.** Procédé selon les revendications 1 à 5, caractérisé en ce que l'on utilise de 3 à 20, de préférence de 5 à 12 parties en volume de mélange d'isomères II et/ou IIa par partie en poids de 2,5-di-(phénylamino)-3,6-dihydrotéréphtalate de dialkyle.

**7.** Procédé de préparation d'une quinacridone, caractérisé en ce que l'on oxyde par des moyens usuels la 6,13-dihydroquinacridone obtenue selon les revendications 1 à 6.

**8.** Procédé de préparation de la $\gamma$-quinacridone, caractérisé en ce que l'on traite la 6,13-dihydroquinacridone obtenue selon revendications 1 à 6 par un mélange essentiellement anhydre d'un alcool en $C_1$-$C_4$, de préférence le méthanol, et d'un alcali, de préférence NaOH, après quoi on oxyde.

**9.** Procédé selon les revendications 7 et 8, caractérisé en ce que l'on oxyde par l'air.